# EUROPÄISCHE PATENTSCHRIFT

(11) **EP 0 366 609 B1**
(45) Veröffentlichungstag und Bekanntmachung des Hinweises auf die Patenterteilung: **27.04.1994**
(21) Anmeldenummer: 89810787.5
(22) Anmeldetag: 17.10.1989
(51) Int. Cl.: A61K 31/44, A61K 31/415, A61K 31/495, A61K 31/535, A61K 31/55

(54) **Verwendung von bicyclischen Imidazol-Verbindungen zur Behandlung von Hyperaldosteronismus**
Use of bicyclic imidazole derivatives in treating hyperaldosteronism
Emploi de dérivés bicycliques de l'imidazole pour le traitement de l'hyperaldostéronisme

(30) Priorität: 26.10.1988 CH 3994/88
(43) Veröffentlichungstag der Anmeldung: 02.05.1990
(73) Patentinhaber: CIBA-GEIGY AG, 4002 Basel (CH)
(72) Erfinder: Häusler, Albert, Dr., CH-4132 Muttenz (CH); Bhatnagar, Ajay, Dr., CH-4052 Basle (CH)

(56) Entgegenhaltungen:
- EP-A- 0 165 904
- JOURNAL OF ENDOCRINOLOGY, Band 119, Supplement, Zusammenfassung Nr. 123, Novemeber 1988, Seite 123; M. DOWSETT et al.: "Dose-related endocrine study of aromatase ihibitor CGS 16949A"
- JOURNAL OF CLINICAL ENDOCRINOLOGY AND METABOLISM, Band, 69, Nr. 4, Oktober 1989, Seiten 896-901, The Endocrine Society, US; S.W.J. LAMBERTS et al.: The new aromatase inhibitor CGS-16949A suppresses aldosterone and cortisol production by human adrenal cells in vitro"
- J. STEROID BIOCHEM., Band 34, Nrs. 1-6, 1989, Seiten 567-570, Pergamon Press plc, GB; A. HÄUSLER et al.: "Evidence that corticosterone is not an obligatory intermediate in aldosterone biosynthesis in the rat adrenal"
- JOURNAL OF CLINICAL ENDOCRINOLOGY AND METABOLISM, Band 68, Nr. 1, 1989, Januar 1989, Seiten 99-106, The Endocrine Society, US; R.J. SANTEN et al.: "Inhibition of aromatase with CGS 16949A in postmenopausal women"
- J. STEROID. BIOCHEM., Band 31, Nr. 1, 1988, Seiten 137-146, GB; M.A. SHAW et al.: "Aminoglutethimide and ketoconazole: Historical perspectives and future prospects"
- CANCER RESEARCH, Band 48, Nr. 4, Februar 1988, Seiten 834-838; K. SCHIEWECK et al: "CGS 16949A, a new nonsteroidal aromatase inhibitor: Effects on hormone-dependent and -independent tumor in vivo"
- J. MED. CHEM., Band 34, Nr. 2, 1991, Seiten 725-736, American Chemical Society; L.J. BROWNE et al.: "Fadrozole hydrochloride: A potent, selecive, nonsteroidal inhibitor of aromatase for the treatment of estrogen-dependent disease"
- STEROIDS, Band 50, Nrs. 1-3, 1987, Seiten 147-161, R.E. STEELE et al.: "In vitro and in vivo studies demonstrating potent and selective estrogen inhibition with the nonsteroidal aromatase inhibitor CGS 16949A"

## Beschreibung

Die vorliegende Erfindung betrifft die Verwendung von bicyclischen Imidazol-Verbindungen zur Behandlung von Hyperaldosteronismus, bzw. die Verwendung von bicyclischen Imidazol-Verbindungen zur Herstellung von pharmazeutischen Präparaten für die Behandlung von Hyperaldosteronismus.

Hyperaldosteronismus steht für Erkrankungen, die durch erhöhte Aldosteronspiegel gekennzeichnet sind. Letztere können z.B. auf eine erhöhte Produktion von Aldosteron zurückgehen (primärer Hyperaldosteronismus, Conn-Syndrom) oder auch andere Ursachen haben (sekundärer Hyperaldosteronismus). Solche Erkrankungen können sowohl im Zusammenhang mit als auch ohne erhöhten Blutdruck (Hypertonie) und dessen klinische Anzeichen bzw. durch ihn verursachte Organschäden auftreten. Weiterhin können solche Erkrankungen sowohl im Zusammenhang mit als auch ohne Elektrolytverlust und dessen Begleitsymptome, sowohl mit als auch ohne Alkalose und den dadurch verursachten Parästhesien und Tetanien, und sowohl mit als auch ohne Hypernatriämie auftreten.

Bei Patienten mit primärem Hyperaldosteronismus werden nach Conn (Auswertung von 145 Fällen) vor allem folgende Symptome beobachtet (Häufigkeit in Klammern angegeben), aus Stegglin und Siegenthaler, Differentialdiagnose innerer Krankheiten, Thieme, Stuttgart 1980: Hypertonie (100 %), Hypokaliämie (90 %), Proteinurie (85 %), Hyposthenurie (80 %), EKG-Veränderungen (80 %), Muskelschwäche (73 %), Polyurie (72 %), Hypernatriämie (65 %), Kopfschmerzen (51 %), Retinopathie (50 %), Polydipsie (46 %), Kardiomegalie (41 %), Parästhesien (24 %), Sehstörungen (21 %), intermittierende Lähmungen (21 %) und intermittierende Tetanie (21 %).

In der Europäischen Patentanmeldung Nr. 165 904 werden bicyclische Imidazol-Verbindungen der Formel I 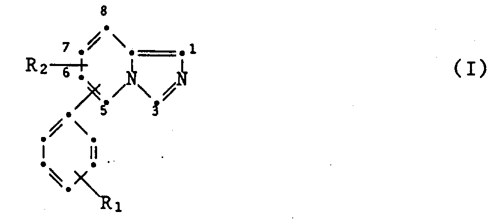
beschrieben, worin R₁ Wasserstoff, Niederalkyl, substituiertes Niederalkyl, Nitro, Halogen, freies, verethertes oder verestertes Hydroxy, freies, verethertes, oxidiert-verethertes oder verestertes Mercapto, unsubstituiertes, mono- oder disubstituiertes Amino, Ammonio, freies oder funktionell abgewandeltes Sulfo, freies oder funktionell abgewandeltes Formyl, C₂-C₂₀-Acyl oder freies oder funktionell abgewandeltes Carboxy bedeutet; und R₂ für Wasserstoff, Niederalkyl, substituiertes Niederalkyl, Halogen; freies, verethertes oder verestertes Hydroxy; freies, verethertes, oxidiert-verethertes oder verestertes Mercapto; freies oder funktionell abgewandeltes Carboxy oder Acyl steht; sowie die 7,8-Dihydro-derivate davon; und ferner Verbindungen der Formel I* 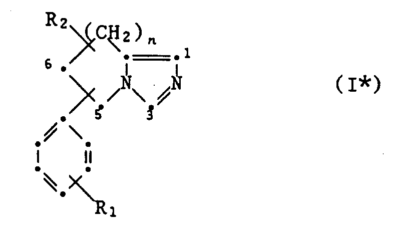
worin n 0, 1, 2, 3 oder 4 bedeutet, und R₁ und R₂ wie oben unter Formel I definiert sind; wobei die beiden Substituenten C₆H₄-R₁ und R₂ an jedem der gesättigten Kohlenstoffatome des gesättigten Rings angeknüpft sein können, entweder beide am selben Kohlenstoffatom oder beide an verschiedenen Kohlenstoffatomen; und Stereoisomere, Mischungen von Stereoisomeren und pharmazeutisch verwendbare Salze davon.

Gemäss oben genannter Patent-Publikationsschrift hemmen die Verbindungen die Aromatase und können daher zur Behandlung von Krankheiten, die auf eine Aromatasehemmung ansprechen, verwendet werden. Als solche Krankheiten werden erwähnt: Gynäkomastie und Oestrogen-abhängige Krankheiten einschliesslich Oestrogen-abhängigem Brustkrebs.

Weiterhin ist z.B. aus der Europäischen Patentanmeldung Nr. 114 573 von einigen der Verbindungen der Formel I und I* bekannt, dass sie auch die Thromboxan-Synthetase hemmen und daher zur Behandlung von Krankheiten, die auf eine Hemmung der Thromboxan-Synthetase ansprechen, wie kardiovaskuläre Krankheiten, z.B. Thromboembolie, geeignet sind.

Wir haben nun gefunden, dass die Verbindungen der Formel I und I*, worin R₁, R₂ und n die obigen Bedeutungen haben, und ihre pharmazeutisch verwendbaren, nicht-toxischen Salze bei Säugern einschliesslich Menschen die Aldosteronausschüttung hemmen und daher zur Behandlung von Hyperaldosteronismus verwendet werden können.

Pharmazeutisch verwendbare, nicht-toxische Säureadditionssalze sind in erster Linie diejenigen mit geeigneten anorganischen oder organischen Säuren, z.B. starken Mineralsäuren, wie Salzsäure, Schwefelsäure oder Phosphorsäure, oder organischen Säuren, insbesondere aliphatischen oder aromatischen Carbon- oder Sulfonsäuren, z.B. Ameisen-, Essig-, Propion-, Bernstein-, Glykol-, Milch-, Hydroxybernstein-, Wein-, Zitronen-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl-, Pamoe-, Glukon-, Nikotin-, Methansulfon-, Ethansulfon-, Halogenbenzolsulfon-, p-Toluolsulfon-, Naphthalinsulfon-, Sulfanil- oder Cyclohexylsulfaminsäure; oder mit anderen sauren organischen Substanzen, z.B. Ascorbinsäure. Ferner können pharmazeutisch verwendbare Salze auch z.B. mit Aminosäuren, wie Arginin oder Lysin, gebildet werden.

Verbindungen der Erfindung, die saure Gruppen enthalten, z.B. eine freie Carboxy- oder Sulfogruppe, können auch pharmazeutisch verwendbare Metall- oder Ammoniumsalze bilden, wie etwa Alkalimetall- oder Erdalkalimetallsalze, z.B. Natrium-, Kalium-, Magnesium- oder Calciumsalze, ferner Ammoniumsalze, die von Ammoniak oder geeigneten organischen Aminen abgeleitet sind. Hier kommen insbesondere aliphatische, cycloaliphatische, cycloaliphatisch-aliphatische oder araliphatische primäre, sekundäre oder tertiäre Mono-, Di- oder Polyamine in Frage, wie Niederalkylamine, z.B. Di- oder Triethylamin, Hydroxy-niederalkylamine, z.B. 2-Hydroxyethyl-amin, Bis-(2-hydroxyethyl)-amin oder Tris-(2-hydroxyethyl)-amin, basische aliphatische Ester oder Carbonsäuren, z.B. 4-Aminobenzoesäure-2-diethyl-aminoethylester, Niederalkylenamine, z.B. 1-Ethylpiperidin, Cycloalkyl-amine, z.B. Dicyclohexylamin, Benzylamine, z.B. N,N'-Dibenzylethylendiamin; ferner heterocyclische Basen, etwa vom Pyridintyp, z.B. Pyridin, Collidin oder Chinolin.

Bei Gegenwart mehrerer saurer oder basischer Gruppen können Mono- oder Polysalze gebildet werden. Erfindungsgemässe Verbindungen, die eine saure und eine basische Gruppe aufweisen, können ferner in Form von inneren Salzen, d.h. als Zwitterionen, vorliegen oder ein Teil des Moleküls kann als inneres Salz vorliegen und ein anderer Teil des Moleküls als normales Salz.

Die die Aldosteronausschüttung hemmende Wirkung der oben genannten Verbindungen der Formel I und I* wird z.B. anhand der folgenden Versuchsanordnungen festgestellt:
1. In-Vitro Methode zur Bestimmung der Hemmung der ACTH-induzierten Aldosteronproduktion in Ratten- oder Mäusen-Nebennieren (ACTH ≙ adrenocorticotropes Hormon): Adulte, männliche Ratten (200-300 g) oder Mäuse (25-35 g) werden dekapitiert, die Nebennieren entnommen und jede Nebenniere in 8 gleichgrosse Fragmente geteilt. Jeweils gleichviele Fragmente werden in mehreren Kölbchen mit einer geeigneten Pufferlösung (Krebs-Ringer-Bikarbonat-Puffer pH 7.4) unter Zusatz von ACTH ohne Testsubstanz (Kontrollwert) bzw. mit verschiedenen Konzentrationen der Testsubstanz 2 Stunden bei 37°C inkubiert. Nach der Inkubation wird das Inkubationsmedium von allfälligen Gewebestücken durch Zentrifugation getrennt. Der Aldosterongehalt des Inkubationsmediums wird mittels eines spezifischen Radioimmunoassays gemessen und als Aldosteronproduktion pro 2 Stunden ausgedrückt. Die Hemmwirkung der Testsubstanzen drückt sich in einer Senkung der Aldosteronproduktion pro 2 Stunden aus und wird zum Beispiel in Prozent (des Kontrollwertes) angegeben. Die Verbindungen der Formel I bewirken eine Senkung der Aldosteronproduktion ab einer Konzentration von etwa 0.1 µM (≙ 100 nM).
2. Die Verbindungen der Formel I und I* hemmen sehr effektiv, nämlich bereits bei einer Konzentration von ca. 100 nM oder bei noch niedrigeren Konzentrationen, die durch Angiotensin-II-stimulierte Aldosteronausschüttung von adrenokortikalen Zellen, die aus einem Adenoma eines Patienten, welcher an Conn-Syndrom (≙ primärer Hyperaldosteronismus) erkrankt ist, präpariert werden.

Die vorliegende Erfindung betrifft deshalb die Verwendung der Verbindungen der Formel I und I*, worin R₁, R₂ und n die obigen Bedeutungen haben, und ihrer pharmazeutisch verwendbaren, nicht-toxischen Salze zur Behandlung von Hyperaldosteronismus, und zwar in Tagesdosen von etwa 5 mg bis etwa 30 mg, bezogen auf ein Individuum von etwa 70 kg Körpergewicht, wobei Einzeldosen von etwa 2 bis etwa 20 mg verabreicht werden.

Die Erfindung betrifft ferner ein Verfahren zur Behandlung von Hyperaldosteronismus unter Verwendung von Verbindungen der Formel I bzw. I*, worin R₁, R₂ und n die obigen Bedeutungen haben, und ihren pharmazeutisch verwendbaren, nicht-toxischen Säureadditionssalzen, sowie die Verwendung von solchen Verbindungen zur Herstellung von pharmazeutischen Präparaten für die Behandlung von Hyperaldosteronismus.

Die vor- und nachstehend zur Definition der Verbindungen der Formel I und I* verwendeten Allgemeinbegriffe haben die folgenden Bedeutungen:
Organische Reste, die mit dem Ausdruck "nieder" bezeichnet sind, enthalten gewöhnlich bis und mit 7, vorzugsweise bis und mit 4, Kohlenstoffatome.

Die Verbindungen der Formel I* sowie bestimmte 7,8-Dihydroderivate der Formel I enthalten wenigstens ein asymmetrisches Kohlenstoffatom. Sie können als R- oder S-Enantiomere sowie als enatiomere Mischungen davon, etwa als Racemat, auftreten. Die Formel I und I* umfasst alle diese Formen, auch alle weiteren Isomere, und Mischungen von wenigstens 2 Isomeren, z.B. Diastereomerengemische oder Enantiomerengemische, die dann vorliegen können, wenn ein oder mehrere weitere asymmetrischen Zentren im Molekül vorliegen.

Niederalkyl ist z.B. n-Propyl, Isopropyl, n-Butyl, Isobutyl, sek-Butyl oder tert-Butyl, ferner n-Pentyl, Isopentyl, Neopentyl, n-Hexyl, Isohexyl oder n-Heptyl, vorzugsweise jedoch Ethyl und im besonderen Methyl.

Substituiertes Niederalkyl R₁ ist vorzugsweise substituiert durch Hydroxy, verethertes Hydroxy, z.B. Niederalkoxy, verestertes Hydroxy, z.B. Niederalkanoyloxy, Acyl, z.B. Niederalkanoyl, Amino, mono- oder disubstituiertes Amino, z.B. Niederalkylamino oder Diniederalkylamino, Halogen, vorzugsweise Fluor, freies oder funktionell abgewandeltes Sulfo, vorzugsweise Sulfo, oder freies oder funktionell abgewandeltes Carboxy, z.B. Carboxy, Niederalkoxycarbonyl, Carbamoyl oder Cyan.

Substituiertes Niederalkyl R₂ ist vorzugsweise substituiert durch Aryl oder freies oder funktionell abgewandeltes Carboxy, insbesondere Carboxy oder Niederalkoxycarbonyl.

Halogen ist z.B. Brom oder Jod, vorzugsweise Fluor und insbesondere Chlor.

Verethertes Hydroxy ist insbesondere Niederalkoxy, ferner z.B. Aryloxy oder Aryl-niederalkoxy. Verestertes Hydroxy ist z.B. Acyloxy, vorzugsweise Niederalkanoyloxy, kann aber z.B. auch Aroyloxy oder Niederalkoxycarbonyloxy bedeuten.

Verethertes Mercapto ist im besonderen Niederalkylthio, kann aber auch z.B. Arylthio oder Aryl-niederalkylthio bedeuten. Oxidiert-verethertes Mercapto ist z.B. Arylsulfinyl oder Arylsulfonyl, und insbesondere Niederalkylsulfinyl oder Niederalkylsulfonyl. Verestertes Mercapto ist z.B. Acylthio, z.B. Niederalkanoylthio.

Monosubstituiertes Amino ist im besonderem Niederalkylamino, bedeutet ferner z.B. Arylamino, Aryl-niederalkylamino oder Acylamino, insbesondere Niederalkanoylamino, kann aber auch z.B. Aroylamino sein.

Disubstituiertes Amino ist im besonderem Diniederalkylamino, und kann ferner z.B. Niederalkylenamino; Oxa-, Thia- oder Aza-niederalkylenamino bedeuten, wobei im letzteren Rest das Azastickstoffatom gegebenenfalls z.B. durch einen Kohlenwasserstoffrest, wie etwa Niederalkyl, substituiert ist. Beispiele für diese Reste sind N-Morpholino, N-Thiomorpholino oder gegebenenfalls in 4-Stellung durch Niederalkyl substituiertes N-Piperazino.

Ammonio umfasst z.B. quaternäre Ammoniumsalze, die sich von den entsprechenden obengenannten disubstituierten Aminogruppen ableiten und die als quaternären Substituenten z.B. gegebenenfalls substituiertes Niederalkyl, vorzugsweise Niederalkyl, Hydroxy-, Halogen- oder Arylniederalkyl enthalten. Im besonderen bedeutet Ammonio Triniederalkylammonio, z.B. Trimethylammonio. Die Ammoniumsalze entsprechen den unten definierten Salzen, insbesondere den Salzen, die als pharmazeutisch verwendbare, nicht-toxische Säureadditionsalze angegeben sind, und vor allem den Salzen, die mit Halogenwasserstoffsäuren, Schwefelsäure oder Phosphorsäure gebildet werden.

Freies oder funktionell abgewandeltes Sulfo ist z.B. Sulfo (-SO₃H), verestertes Sulfo, z.B. Niederalkoxysulfonyl, amidiertes Sulfo, z.B. Sulfamoyl, Niederalkylsulfamoyl oder Diniederalkylsulfamoyl, oder ein Sulfonylhalogenid, z.B. Sulfonylchlorid; und ist vorzugsweise Sulfo oder Sulfamoyl.

Freies oder funktionell abgewandeltes Formyl ist vorzugsweise Formyl oder Iminomethyl (-CH=NH), welches am Stickstoff durch freies, verethertes oder verestertes Hydroxy, z.B. Hydroxy, Niederalkoxy oder Niederalkanoyloxy, durch Niederalkyl, Aryl oder Amino substituiert sein kann; ferner aber auch z.B. ein Acetal, wie etwa ein Diniederalkylacetal, z.B. Dimethylacetal.

Acyl, das gewöhnlich 1-20 Kohlenstoffatome enthält, bedeutet das entsprechende Radikal einer Carbonsäure, vorzugsweise Aroyl oder Halogen-C₂-C₇-alkanoyl, und im besonderen Niederalkanoyl. C₁-Alkanoyl entspricht Formyl.

Freies oder funktionell abgewandeltes Carboxy ist z.B. Carboxy, verestertes Carboxy, vorzugsweise Niederalkoxycarbonyl; amidiertes Carboxy, vorzugsweise Carbamoyl, Niederalkylcarbamoyl, Diniederalkylcarbamoyl oder Hydroxycarbamoyl; oder Cyan. Weiterhin sind z.B. heterocyclische Derivate von Carboxy umfasst, vorzugsweise 5-Tetrazolyl oder unsubstituiertes oder durch Niederalkyl substituiertes 4,5-Dihydro-2-oxazolyl.

Aryl als solches oder innerhalb von Resten wie z.B. Aryloxy, Aryl-niederalkylthio, Arylsulfonyl, Arylamino usw. bedeutet z.B. 1- oder 2-Naph-thyl, vorzugsweise Phenyl, das durch z.B. Niederalkyl, Niederalkoxy und/oder Halogen substituiert, vorzugsweise monosubstituiert, ist, und in erster Linie Phenyl. Aroyl als solches oder innerhalb von Resten wie Aroyloxy usw. bedeutet Arylcarbonyl, im besonderen Benzoyl.

Niederalkoxy ist vorzugsweise Methoxy oder Ethoxy, ferner z.B. n-Propoxy, Isopropoxy, n-Butoxy, Isobutoxy oder tert-Butoxy.

Niederalkanoyloxy ist z.B. Formyloxy, Acetoxy, Propionyloxy oder Pivaloyloxy.

Niederalkanoyl ist z.B. Formyl, Acetyl, Propionyl oder Pivaloyl. Halogen-C₂-C₇-Alkanoyl ist vorzugsweise Trifluoracetyl. Niederalkanoylamino ist vorzugsweise Acetylamino oder Propionylamino, kann aber z.B. auch Formylamino sein.

Niederalkoxycarbonyl ist vorzugsweise Methoxycarbonyl oder Ethoxycarbonyl. Niederalkoxycarbonyloxy ist z.B. Methoxycarbonyloxy oder Ethoxycarbonyloxy.

Niederalkylamino bedeutet z.B. Methylamino, Ethylamino, n-Propylamino oder Isopropylamino. Diniederalkylamino ist z.B. Dimethylamino, Ethylmethylamino oder Diethylamino. Niederalkylenamino enthält z.B. 2 bis 7, vorzugsweise 4 bis 6, Ringkohlenstoffatome und ist z.B. N-Pyrrolidino oder N-Piperidino.

Niederalkylthio ist z.B: Methylthio, Ethylthio, n-Propylthio oder Isopropylthio, während Niederalkylsulfinyl z.B. Methylsulfinyl bedeutet und Niederalkylsulfonyl z.B. für Methylsulfonyl oder Ethylsulfonyl steht. Niederalkanoylthio ist vorzugsweise Formylthio oder Acetylthio.

Niederalkoxysulfonyl ist z.B. Methoxysulfonyl oder Ethoxysulfonyl. Niederalkylsulfamoyl ist z.B. N-Methyl- oder N-Ethylsulfamoyl, während Diniederalkylsulfamoyl z.B. Dimethyl- oder Diethylsulfamoyl bedeutet.

Niederalkylcarbamoyl ist z.B. N-Methylcarbamoyl oder N-Ethylcarbamoyl, während Diniederalkylcarbamoyl z.B. Dimethyl- oder Diethylcarbamoyl bedeutet.

Vorzugsweise betrifft die Erfindung die Verwendung von Verbindungen der Formel I, worin R₁ Wasserstoff, Niederalkyl; Niederalkyl, das durch Hydroxy, Niederalkoxy, Niederalkanoyloxy, Niederalkanoyl, Amino, Niederalkylamino, Diniederalkylamino, Halogen, Sulfo, Carboxy, Niederalkoxycarbonyl, Carbamoyl oder Cyan substituiert ist; Nitro, Halogen, Hydroxy, Niederalkoxy, Niederalkanoyloxy, Phenylsulfonyloxy, Niederalkylsulfonyloxy, Mercapto, Niederalkylthio, Niederalkylsulfinyl, Niederalkylsulfonyl, Niederalkanoylthio, Amino, Niederalkylamino, Diniederalkylamino, Niederalkylenamino, N-Morpholino, N-Thiomorpholino, gegebenenfalls in 4-Stellung Niederalkyl-substituiertes N-Piperazino, Triniederalkylammonio, Sulfo, Niederalkoxysulfonyl, Sulfamoyl, Niederalkylsulfamoyl, Diniederalkylsulfamoyl, Formyl; Iminomethyl, das gegebenenfalls am Stickstoff durch Hydroxy, Niederalkoxy, Niederalkanoyloxy, Niederalkyl, Phenyl oder Amino substituiert ist; C₂-C₇-Alkanoyl, Benzoyl, Carboxy, Niederalkoxycarbonyl, Carbamoyl, Niederalkylcarbamoyl, Diniederalkylcarbamoyl, Cyan, 5-Tetrazolyl, gegebenenfalls Niederalkyl-substituiertes 4,5-Dihydro-2-oxazolyl oder Hydroxycarbamoyl bedeutet; und R₂ für Wasserstoff, Niederalkyl, Phenyl-niederalkyl, Carboxy-niederalkyl, Niederalkoxycarbonyl-niederalkyl, Halogen, Hydroxy, Niederalkoxy, Niederalkanoyloxy, Mercapto, Niederalkylthio, Phenyl-niederalkylthio, Phenylthio, Niederalkanoylthio, Carboxy, Niederalkoxycarbonyl oder Niederalkanoyl steht; den 7,8-Dihydroderivaten davon; oder den Verbindungen der Formel I*, worin n für 0, 1, 2, 3 oder 4 steht; und R₁ und R₂ wie oben unter Formel I definiert sind; wobei der Phenylring in den Resten Phenylsulfonyloxy, Phenyliminomethyl, Benzoyl, Phenyl-niederalkyl, Phenyl-niederalkylthio und Phenylthio unsubstituiert oder durch Niederalkyl, Niederalkoxy oder Halogen substituiert sein kann; wobei in einer Verbindung der Formel I* die beiden Substituenten C₆H₄-R₁ und R₂ an jedem der gesättigten Kohlenstoffatome des gesättigten Rings angeknüpft sein können, entweder beide am selben Kohlenstoffatom oder beide an verschiedenen Kohlenstoffatomen; Stereoisomeren, Mischungen von Stereoisomeren oder pharmazeutisch verwendbaren Salzen davon zur Behandlung von Hyperaldosteronismus, bzw. ein Verfahren zur Behandlung von Hyperaldosteronismus unter Verwendung von solchen Verbindungen, bzw. die Verwendung dieser Verbindungen zur Herstellung von Präparaten zur Behandlung von Hyperaldosteronismus.

Im besonderen betrifft die Erfindung die Verwendung von Verbindungen der Formel I, worin R₁ Niederalkyl; Niederalkyl, das durch Hydroxy, Amino, Diniederalkylamino, 1-5 Fluoratome, Carboxy, Niederalkoxycarbonyl, Carbamoyl oder Cyan substituiert ist; Nitro, Halogen, Hydroxy, Niederalkoxy, Amino, Niederalkylamino, Diniederalkylamino, Sulfo, Sulfamoyl, Formyl, Iminomethyl; Iminomethyl, das am Stickstoff durch Hydroxy, Niederalkoxy, Niederalkanoyloxy, Niederalkyl oder Phenyl substituiert ist; Carboxy, Niederalkoxycarbonyl, Carbamoyl, Niederalkylcarbamoyl, Diniederalkylcarbamoyl oder Cyan bedeutet; und R₂ für Wasserstoff, Niederalkyl, Niederalkoxy oder Halogen steht; oder Verbindungen der Formel I*, worin n für 1, 2 oder 3 steht; R₁ wie oben unter Formel I definiert ist und R₂ Wasserstoff, Niederalkyl, Phenyl-niederalkyl, Carboxy-niederalkyl, Niederalkoxycarbonyl-niederalkyl, Halogen, Niederalkoxy, Niederalkylthio, Phenyl-niederalkylthio, Phenylthio, Carboxy, Niederalkoxycarbonyl oder Niederalkanoyl bedeutet; wobei in einer Verbindung der Formel I* die beiden Substituenten C₆H₄-R₁ und R₂ an jedem der gesättigten Kohlenstoffatome des gesättigten Rings angeknüpft sein können, entweder beide am selben Kohlenstoffatom oder beide an verschiedenen Kohlenstoffatomen; Stereoisomeren, Mischung von Stereoisomeren oder pharmazeutisch verwendbaren Salzen davon zur Behandlung von Hyperaldosteronismus, bzw. ein Verfahren zur Behandlung von Hyperaldosteronismus unter Verwendung von solchen Verbindungen, bzw. die Verwendung dieser Verbindungen zur Herstellung von Präparaten zur Behandlung von Hyperaldosteronismus.

In erster Linie betrifft die Erfindung die Verwendung von Verbindungen der Formel I, worin R₁ Niederalkyl, Hydroxy-niederalkyl, Halogen, Amino, Formyl, Carboxy, Niederalkoxycarbonyl, Carbamoyl, N-Niederalkylcarbamoyl oder Cyan bedeutet; und R₂ Wasserstoff ist; oder Verbindungen der Formel I*, worin n für 1, 2 oder 3 steht; R₁ wie oben unter Formel I definiert ist und R₂ Wasserstoff, Niederalkylthio, Niederalkoxycarbonyl, Phenyl-niederalkyl, Carboxy-niederalkyl oder Niederalkoxycarbonyl-niederalkyl bedeutet; wobei in einer Verbindung der Formel I* die beiden Substituenten C₆H₄-R₁ und R₂ an jedem der gesättigten Kohlenstoffatome des gesättigten Rings angeknüpft sein können, entweder beide am selben Kohlenstoffatom oder beide an verschiedenen Kohlenstoffatomen; Stereoisomeren, Mischungen von Stereoisomeren oder pharmazeutisch verwendbaren Salze davon zur Behandlung von Hyperaldosteronismus, bzw. ein Verfahren zur Behandlung von Hyperaldosteronismus unter Verwendung von solchen Verbindungen, bzw. die Verwendung dieser Verbindungen zur Herstellung von Präparaten zur Behandlung von Hyperaldosteronismus.

Die Erfindung betrifft insbesondere die Verwendung einer der folgenden Verbindungen,
(a) 5-(p-Cyanphenyl)imidazo[1,5-a]pyridin,
(b) 5-(p-Ethoxycarbonylphenyl)imidazo[1,5-a]pyridin,
(c) 5-(p-Carboxyphenyl)imidazo[1,5-a]pyridin,
(d) 5-(p-tert-Butylaminocarbonylphenyl)imidazo[1,5-a)pyridin,
(e) 5-(p-Ethoxycarbonylphenyl)-5,6,7,8-tetrahydroimidazo[1,5-a]pyridin,
(f) 5-(p-Carboxyphenyl)-5,6,7,8-tetrahydroimidazo[1,5-a]pyridin,
(g) 5-(p-Carbamoylphenyl)-5,6,7,8-tetrahydroimidazo[1,5-a]pyridin,
(h) 5-(p-Tolyl)-5,6,7,8-tetrahydroimidazo[1,5-a]pyridin,
(i) 5-(p-Hydroxymethylphenyl)imidazo[1,5-a]pyridin,
(j) 5-(p-Cyanphenyl)-7,8-dihydroimidazo[1,5-a]pyridin,
(k) 5-(p-Bromphenyl)-5,6,7,8-tetrahydroimidazo[1,5-a]pyridin,
(l) 5-(p-Hydroxymethylphenyl)-5,6,7,8-tetrahydroimidazo[1,5-a]pyridin,
(m) 5-(p-Formylphenyl)-5,6,7,8-tetrahydroimidazo[1,5-a]pyridin,
(n) 5-(p-Cyanphenyl)-5-methylthio-5,6,7,8-tetrahydroimidazo[1,5-a]pyridin,
(o) 5-(p-Cyanphenyl)-5-ethoxycarbonyl-5,6,7,8-tetrahydroimidazo[1,5-a]pyridin,
(p) 5-(p-Aminophenyl)-5,6,7,8-tetrahydroimidazo[1,5-a]pyridin,
(q) 5-(p-Formylphenyl)imidazo[1,5-a]pyridin,
(r) 5-(p-Carbamoylphenyl)imidazo[1,5-a]pyridin,
(s) 5H-5-(4-tert-Butylaminocarbonylphenyl)-6,7-dihydropyrrolo[1,2-c]imidazol,
(t) 5H-5-(4-Cyanphenyl)-6,7-dihydropyrrolo[1,2-c]imidazol,
(u) 5H-5-(4-Cyanphenyl)-6,7,8,9-tetrahydroimidazo[1,5-a]azepin,
(v) 5-(4-Cyanphenyl)-6-ethoxycarbonylmethyl-5,6,7,8-tetrahydroimidazo[1,5-a]pyridin,
(w) 5-(4-Cyanphenyl)-6-carboxymethyl-5,6,7,8-tetrahydroimidazo[1,5-a]pyridin,
(x) 5-Benzyl-5-(4-Cyanphenyl)-5,6,7,8-tetrahydroimidazo[1,5-a]pyridin,
(y) 7-(p-Cyanphenyl)-5,6,7,8-tetrahydroimidazo[1,5-a]pyridin,
(z) 7-(p-Carbamoylphenyl)-5,6,7,8-tetrahydroimidazo[1,5-a]pyridin,
oder einem pharmazeutisch verwendbaren, nicht-toxischen Salz davon, zur Behandlung von Hyperaldosteronismus, bzw. ein Verfahren zur Behandlung von Hyperaldosteronismus unter Verwendung von solchen Verbindungen, bzw. die Verwendung dieser Verbindungen zur Herstellung von Präparaten zur Behandlung von Hyperaldosteronismus.

Die Erfindung betrifft vor allen Dingen die Verwendung von 5-(p-Cyanphenyl)-5,6,7,8-tetrahydroimidazo[1,5-a]pyridin oder einem pharmazeutisch verwendbaren, nicht-toxischen Säureadditionssalz davon zur Behandlung von Hyperaldosteronismus, bzw. ein Verfahren zur Behandlung von Hyperaldosteronismus unter Verwendung von solchen Verbindungen, bzw. die Verwendung dieser Verbindungen zur Herstellung von Präparaten zur Behandlung von Hyperaldosteronismus. Das Hydrochlorid der genannten Verbindung hemmt die ACTH-induzierte Aldosteronproduktion in Ratten- und Mäusenebennieren (Testanordnung 1) mit einem IC₅₀-Wert von 1,0 µM. Es hemmt ferner die Angiotensin-II-stimulierte Aldosteronausschüttung von adrenokortikalen Zellen eines an primärem Hyperaldosteronismus erkrankten Patienten (Testanordnung 2) noch maximal bei einer Konzentration von 100 nM.

Die Erfindung betrifft ferner die Verwendung der optischen Antipoden der oben genannten Verbindung, (a) (-)-5-(p-Cyanphenyl)-5,6,7,8-tetrahydroimidazo[1,5-a]pyridin und (b) (+)-5-(p-Cyanphenyl)-5,6,7,8-tetrahydroimidazo[1,5-a]pyridin, oder von einem pharmazeutisch verwendbaren, nichttoxischen Säureadditionssalz davon zur Behandlung von Hyperaldosteronismus, bzw. ein Verfahren zur Behandlung von Hyperaldosteronismus unter Verwendung von solchen Verbindungen, bzw. die Verwendung dieser Verbindungen zur Herstellung von Präparaten zur Behandlung von Hyperaldosteronismus.

Ueberraschenderweise wurde gefunden, dass der oben erwähnte (+)-Antipode in vitro und in vivo eine viel grössere Spezifität aufweist als das entsprechende Racemat.

Folglich betrifft die Erfindung in allererster Linie die Verwendung von (+)-5-(p-Cyanphenyl)-5,6,7,8-tetrahydroimidazo[1,5-a]pyridin, oder einem pharmazeutisch verwendbaren, nicht-toxischen Säureadditionssalz davon zur Behandlung von Hyperaldosteronismus, bzw. ein Verfahren zur Behandlung von Hyperaldosteronismus unter Verwendung von solchen Verbindungen, bzw. die Verwendung dieser Verbindungen zur Herstellung von Präparaten zur Behandlung von Hyperaldosteronismus.

Wie oben ausgeführt worden ist, können die Verbindungen der Formel I und I*, worin R₁, R₂ und n die oben genannten Bedeutungen haben, und pharmazeutisch verwendbare, nicht-toxische Salze davon zur Herstellung von pharmazeutischen Präparaten für die Behandlung von Hyperaldosteronismus verwendet werden.

Bei den erfindungsgemäss herstellbaren pharmazeutischen Präparaten handelt es sich um solche zur enteralen, wie peroralen oder rektalen, ferner zur sublingualen, sowie zur parenteralen Verabreichung. Entsprechende Dosiseinheitsformen, insbesondere zur peroralen und/oder sublingualen Verabreichung, z.B. Dragées, Tabletten oder Kapseln, enthalten vorzugsweise von etwa 2 mg bis etwa 20 mg, insbesondere von etwa 2 mg bis etwa 10 mg einer der obgenannten Verbindungen oder eines pharmazeutisch verwendbaren Salzes davon zusammen mit pharmazeutisch verwendbaren Trägerstoffen. Dabei beträgt der Anteil des Wirkstoffs in solchen pharmazeutischen Präparaten zwischen etwa 5 % und etwa 90 %, vorzugsweise zwischen etwa 10 % und etwa 60 %.

Für pharmazeutische Präparate zur oralen Verabreichung sind geeignete Hilfsmittel insbesondere Füllstoffe, wie Zucker, z.B. Lactose, Saccharose, Mannit oder Sorbit, Cellulosepräparate, und/oder Calciumphosphate, z.B. Tricalciumphosphat oder Calciumhydrogenphosphat, Bindemittel, wie Stärke z.B. Mais-, Weizen-, Reis- oder Kartoffelstärke, Gelatine, Traganth, Methylcellulose und/oder Hydroxypropyl-cellulose, Sprengmittel, wie die obgenannten Stärken, Carboxymethylstärke, quervernetztes Polyvinyl-pyrrolidon, Agar, Alginsäure oder ein Salz davon, wie Natriumalginat und/oder Cellulose, z.B. in kristalliner, besonders in mikrokristalliner Form, und/oder Fliessregulier- und Schmiermittel, z.B. Kieselsäure, Talk, Stearinsäure oder Salze davon, wie Magnesium- oder Calciumstearat, Cellulose und/oder Polyethylenglykol.

Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, oder Lacklösungen in geeigneten Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls mit Stabilisatoren, enthalten. In weichen Kapseln ist der Wirkstoff vorzugsweise in geeigneten öligen Hilfsstoffen, wie fetten Oelen, Paraffinöl oder flüssigen Polyethylenglykolen, gelöst oder suspendiert, wobei ebenfalls Stabilisatoren und/oder antibakterielle Mittel zugefügt sein können. Ferner können Kapseln verwendet werden, die leicht zerbissen werden können, um durch die so stattfindene sublinguale Aufnahme des Wirkstoffes eine möglichst rasche Wirkung zu erzielen.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Den pharmazeutischen Präparaten, insbesondere den Tabletten oder Dragée-Ueberzügen, können Farbstoffe oder Pigmente, z.B. zur Identifizierung oder zur Kennzeichnung verschiedener Wirkstoffdosen, beigefügt werden.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die beanspruchte Erfindung wird in den folgenden Beispielen näher beschrieben;

### Beispiel 1: Es werden 10000 Tabletten hergestellt, die je 10 mg des Wirkstoffs, als freie Base, enthalten:

### Zusammensetzung:

| | |
|---|---|
| 5-(p-Cyanphenyl)-5,6,7,8-tetrahydroimidazo-[1,5-a]pyridin-hydrochlorid | 116,33 g |
| Milchzucker | 2535,00 g |
| Maisstärke | 125,00 g |
| Polyethylenglykol 6000 | 150,00 g |
| Magnesiumstearat | 40,00 g |
| Gereinigtes Wasser | quantum satis |

### Verfahren:

Alle pulverigen Bestandteile werden durch ein Sieb mit einer Maschenweite von 0,6 mm gesiebt. Dann wird der Wirkstoff, Milchzucker, Magnesiumstearat und die Hälfte der Stärke in einem geeigneten Mischer vermischt. Die andere Hälfte der Stärke wird in 65 ml Wasser suspendiert un die Suspension zur siedenden Lösung des Polyethylenglykols in 260 ml Wasser gegeben. Die gebildete Paste wird den Pulvern zugesetzt und gegebenenfalls unter Zugabe von mehr Wasser granuliert. Das Granulat wird über Nacht bei 35° getrocknet, durch ein Sieb mit 1,2 mm Maschenweite getrieben und zu Tabletten, welche eine Bruchrille aufweisen, gepresst.

Beispiel 1a: Es wird das Verfahren des Beispiels 1 durchgeführt, mit der einen Aenderung, dass als Wirkstoff 116,33 g (+)-5-(p-Cyanphenyl)-5,6,7,8-tetrahydroimidazo[1,5-a]pyridin-hydrochlorid eingesetzt werden. Man erhält 10000 Tabletten, die je 10 mg des Wirkstoffs, als freie Base, enthalten.

Beispiel 2: Weichgelatinekapseln, enthaltend 20 mg 5-(p-Cyanphenyl)-5,6,7,8-tetrahydroimidazo[1,5-a]pyridin-hydrochlorid können hergestellt werden, indem man 150 mg eines Gemisches, bestehend aus 20,000 mg 5-(p-Cyanphenyl)-5,6,7,8-tetrahydroimidazo[1,5-a]pyridin-hydrochlorid, 0,075 mg 2,6-Di-tert-butyl-4-methylphenol, 0,015 mg Citronensäure, 0,135 mg 1,2-Propylenglycol, 102,775 mg Rüböl, 20,000 mg Soyalecithin und 7,000 mg Wachsmischung, nach an sich bekannten Standardverfahren, insbesondere nach dem R.P. Scherer-Verfahren, zu einer Weichgelatinekapsel verarbeitet. Deren Hülle besteht aus 0,250 mg Ethylparaben (4-Hydroxy-benzoesäureethylester), 0,130 mg Propylparaben (4-Hydroxy-benzoesäure-n-propylester), 25,000 mg Gelatine und 54,600 mg Glycerin.

Beispiel 2a: Es wird das Verfahren des Beispiels 2 durchgeführt, mit der einen Aenderung, dass als Wirkstoff 20,000 mg (+)-5-(p-Cyanphenyl)-5,6,7,8-tetrahydroimidazo[1,5-a]pyridin-hydrochlorid eingesetzt werden. Man erhält Weichgelatinekapseln, die 20 mg dieses Wirkstoffs enthalten.

## Patentansprüche

1. Verwendung von Verbindungen der Formel I 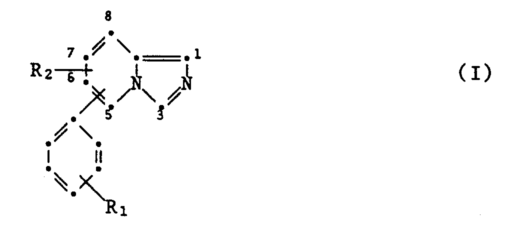 worin R₁ Wasserstoff, Niederalkyl, substituiertes Niederalkyl, Nitro, Halogen, freies, verethertes oder verestertes Hydroxy, freies, verethertes, oxidiert-verethertes oder verestertes Mercapto, unsubstituiertes, mono- oder disubstituiertes Amino, Ammonio, freies oder funktionell abgewandeltes Sulfo, freies oder funktionell abgewandeltes Formyl, C₂-C₂₀-Acyl oder freies oder funktionell abgewandeltes Carboxy bedeutet; und R₂ für Wasserstoff, Niederalkyl, substituiertes Niederalkyl, Halogen; freies, verethertes oder verestertes Hydroxy; freies, verethertes, oxidiert-verethertes oder verestertes Mercapto; freies oder funktionell abgewandeltes Carboxy oder Acyl steht; den 7,8-Dihydroderivaten davon; und Verbindungen der Formel I* 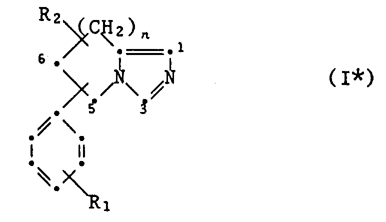 worin n 0, 1, 2, 3 oder 4 bedeutet, und R₁ und R₂ wie oben unter Formel I definiert sind; wobei die beiden Substituenten C₆H₄-R₁ und R₂ an jedem der gesättigten Kohlenstoffatome des gesättigten Rings angeknüpft sein können, entweder beide am selben Kohlenstoffatom oder beide an verschiedenen Kohlenstoffatomen; Stereoisomeren, Mischungen von Stereoisomeren und pharmazeutisch verwendbaren Salzen davon zur Herstellung von pharmazeutischen Präparaten zur Behandlung von Hyperaldosteronismus.

2. Verwendung nach Anspruch 1, dadurch gekennzeichnet, dass man Verbindungen der Formel I, worin R₁ Wasserstoff, Niederalkyl; Niederalkyl, das durch Hydroxy, Niederalkoxy, Niederalkanoyloxy, Niederalkanoyl, Amino, Niederalkylamino, Diniederalkylamino, Halogen, Sulfo, Carboxy, Niederalkoxycarbonyl, Carbamoyl oder Cyan substituiert ist; Nitro, Halogen, Hydroxy, Niederalkoxy, Niederalkanoyloxy, Phenylsulfonyloxy, Niederalkylsulfonyloxy, Mercapto, Niederalkylthio, Niederalkylsulfinyl, Niederalkylsulfonyl, Niederalkanoylthio, Amino, Niederalkylamino, Diniederalkylamino, Niederalkylenamino, N-Morpholino, N-Thiomorpholino, gegebenenfalls in 4-Stellung Niederalkyl-substituiertes N-Piperazino, Triniederalkylammonio, Sulfo, Niederalkoxysulfonyl, Sulfamoyl, Niederalkylsulfamoyl, Diniederalkylsulfamoyl, Formyl; Iminomethyl, das gegebenenfalls am Stickstoff durch Hydroxy, Niederalkoxy, Niederalkanoyloxy, Niederalkyl, Phenyl oder Amino substituiert ist; C₂-C₇-Alkanoyl, Benzoyl, Carboxy, Niederalkoxycarbonyl, Carbamoyl, Niederalkylcarbamoyl, Diniederalkylcarbamoyl, Cyan, 5-Tetrazolyl, gegebenenfalls Niederalkyl-substituiertes 4,5-Dihydro-2-oxazolyl oder Hydroxycarbamoyl bedeutet; und R₂ für Wasserstoff, Niederalkyl, Phenyl-nieder-alkyl, Carboxy-niederalkyl, Niederalkoxycarbonyl-niederalkyl, Halogen, Hydroxy, Niederalkoxy, Niederalkanoyloxy, Mercapto, Niederalkylthio, Phenyl-niederalkylthio, Phenylthio, Niederalkanoylthio, Carboxy, Niederalkoxycarbonyl oder Niederalkanoyl steht; 7,8-Dihydroderivate davon; oder Verbindungen der Formel I*, worin n für 0, 1, 2, 3 oder 4 steht; und R₁ und R₂ wie oben unter Formel I definiert sind; wobei der Phenylring in den Resten Phenylsulfonyloxy, Phenyliminomethyl, Benzoyl, Phenyl-niederalkyl, Phenyl-niederalkylthio und Phenylthio unsubstituiert oder durch Niederalkyl, Niederalkoxy oder Halogen substituiert sein kann; wobei in einer Verbindung der Formel I* die beiden Substituenten C₆H₄-R₁ und R₂ an jedem der gesättigten Kohlenstoffatome des gesättigten Rings angeknüpft sein können, entweder beide am selben Kohlenstoffatom oder beide an verschiedenen Kohlenstoffatomen; Stereoisomere, Mischungen von Stereoisomeren oder pharmazeutisch verwendbare Salze davon anwendet.

3. Verwendung nach Anspruch 1, dadurch gekennzeichnet, dass man Verbindungen der Formel I, worin R₁ Niederalkyl; Niederalkyl, das durch Hydroxy, Amino, Diniederalkylamino, 1-5 Fluoratome, Carboxy, Niederalkoxycarbonyl, Carbamoyl oder Cyan substituiert ist; Nitro, Halogen, Hydroxy,-Niederalkoxy, Amino, Niederalkylamino, Diniederalkylamino, Sulfo, Sulfamoyl, Formyl, Iminomethyl; Iminomethyl, das am Stickstoff durch Hydroxy, Niederalkoxy, Niederalkanoyloxy, Niederalkyl oder Phenyl substituiert ist; Carboxy, Niederalkoxycarbonyl, Carbamoyl, Niederalkylcarbamoyl, Diniederalkylcarbamoyl oder Cyan bedeutet; und R₂ für Wasserstoff, Niederalkyl, Niederalkoxy oder Halogen steht; oder Verbindungen der Formel I*, worin n für 1, 2 oder 3 steht; R₁ wie oben unter Formel I definiert ist und R₂ Wasserstoff, Niederalkyl, Phenyl-niederalkyl, Carboxy-niederalkyl, Niederalkoxycarbonyl-niederalkyl, Halogen, Niederalkoxy, Niederalkylthio, Phenyl-niederalkylthio, Phenylthio, Carboxy, Niederalkoxycarbonyl oder Niederalkanoyl bedeutet; wobei in einer Verbindung der Formel I* die beiden Stibstituenten C₆H₄-R₁ und R₂ an jedem der gesättigten Kohlenstoffatome des gesättigten Rings angeknüpft sein können, entweder beide am selben Kohlenstoffatom oder beide an verschiedenen Kohlenstoffatomen; Stereoisomere, Mischung von Stereoisomeren oder pharmazeutisch verwendbare Salze davon anwendet.

4. Verwendung nach Anspruch 1, dadurch gekennzeichnet, dass man Verbindungen der Formel I, worin R₁ Niederalkyl, Hydroxy-niederalkyl, Halogen, Amino, Formyl, Carboxy, Niederalkoxycarbonyl, Carbamoyl, N-Niederalkylcarbamoyl oder Cyan bedeutet; und R₂ Wasserstoff ist; oder Verbindungen der Formel I*, worin n für 1, 2 oder 3 steht; R₁ wie oben unter Formel I definiert ist und R₂ Wasserstoff, Niederalkylthio, Niederalkoxycarbonyl, Phenyl-niederalkyl, Carboxy-niederalkyl oder Niederalkoxycarbonyl-niederalkyl bedeutet; wobei in einer Verbindung der Formel I* die beiden Substituenten C₆H₄-R₁ und R₂ an jedem der gesättigten Kohlenstoffatome des gesättigten Rings angeknüpft sein können, entweder beide am selben Kohlenstoffatom oder beide an verschiedenen Kohlenstoffatomen; Stereoisomere, Mischungen von Stereoisomeren oder pharmazeutisch verwendbare Salze davon anwendet.

5. Verwendung nach Anspruch 1, dadurch gekennzeichnet, dass man 5-(p-Cyanphenyl)-5,6,7,8-tetrahydroimidaso[1,5-a]pyridin oder ein pharmazeutisch verwendbares, nicht-toxisches Säureadditionssalz davon anwendet.

6. Verwendung nach Anspruch 1, dadurch gekennzeichnet, dass man (+)-5-(p-Cyanphenyl)-5,6,7,8-tetrahydroimidazo[1,5-a]pyridin oder ein pharmazeutisch verwendbares, nicht-toxisches Säureadditionssalz davon anwendet.

7. Verwendung nach Anspruch 1, dadurch gekennzeichnet, dass man (-)-5-(p-Cyanphenyl)-5,6,7,8-tetrahydroimidazo[1,5-a]pyridin oder ein pharmazeutisch verwendbares, nicht-toxisches Säureadditionssalz davon anwendet.

## Claims

1. Use of a compound of formula I 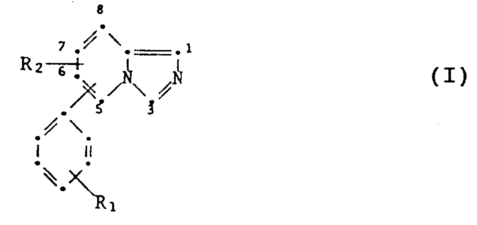 wherein
R₁ is hydrogen, lower alkyl, substituted lower alkyl, nitro, halogen, free, etherified or esterified hydroxy, free, etherified, oxidised-etherified or esterified mercapto, unsubstituted, mono- or di-substituted amino, ammonio, free or functionally modified sulfo, free or functionally modified formyl, C₂-C₂₀acyl or free or functionally modified carboxy; and
R₂ is hydrogen, lower alkyl, substituted lower alkyl, halogen; free, etherified or esterified hydroxy; free, etherified, oxidised-etherified or esterified mercapto; free or functionally modified carboxy or acyl;
or of a 7,8-dihydro derivative thereof; or of a compound of formula I* 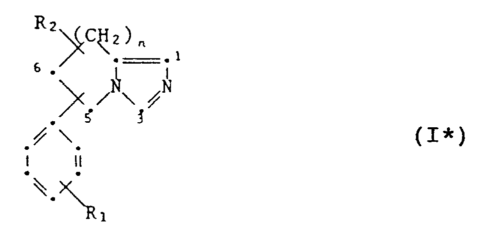 wherein n is O, 1, 2, 3 or 4; and R₁ and R₂ are as defined above for formula I; it being possible for the two substituents C₆H₄-R₁ and R₂ to be attached to each of the saturated carbon atoms of the saturated ring, either both to the same carbon atom or both to different carbon atoms; or of a stereoisomer or mixture of stereoisomers, or of a pharmaceutically acceptable salt thereof, in the preparation of pharmaceutical compositions for the treatment of hyperaldosteronism.

2. Use according to claim 1 of a compound of formula I, wherein
R₁ is hydrogen, lower alkyl; lower alkyl which is substituted by hydroxy, lower alkoxy, lower alkanoyloxy, lower alkanoyl, amino, lower alkylamino, di-lower alkylamino, halogen, sulfo, carboxy, lower alkoxycarbonyl, carbamoyl or cyano; or is nitro, halogen, hydroxy, lower alkoxy, lower alkanoyloxy, phenylsulfonyloxy, lower alkylsulfonyloxy, mercapto, lower alkylthio, lower alkylsulfinyl, lower alkylsulfonyl, lower alkanoylthio, amino, lower alkylamino, di-lower alkylamino, lower alkyleneamino, N-morpholino, N-thiomorpholino, N-piperazino or N-piperazino which is substituted in the 4-position by lower alkyl; or is trilower alkylammonio, sulfo, lower alkoxysulfonyl, sulfamoyl, lower alkylsulfamoyl, di-lower alkylsulfamoyl, formyl; or is iminomethyl which may be substituted at the nitrogen by hydroxy, lower alkoxy, lower alkanoyloxy, lower alkyl, phenyl or amino; or is C₂-C₇alkanoyl, benzoyl, carboxy, lower alkoxycarbonyl, carbamoyl, lower alkylcarbamoyl, di-lower alkylcarbamoyl, cyano, 5-tetrasolyl, unsubstituted or lower alkyl-substituted 4,5-dihydro-2-oxasolyl or hydroxycarbamoyl; and
R₂ is hydrogen, lower alkyl, phenyl-lower alkyl, carboxy-lower alkyl, lower alkoxycarbonyl-lower alkyl, halogen, hydroxy, lower alkoxy, lower alkanoyloxy, mercapto, lower alkylthio, phenyl-lower alkylthio, phenylthio, lower alkanoylthio, carboxy, lower alkoxycarbonyl or lower alkanoyl;
or of a 7,8-dihydro derivative thereof; or of a compound of formula I* wherein n is O, 1, 2, 3 or 4; and R₁ and R₂ are as defined above for formula I; and the phenyl ring in the radicals phenylsulfonyloxy, phenyliminomethyl, benzoyl, phenyl-lower alkyl, phenyl-lower alkylthio and phenylthio may be unsubstituted or substituted by lower alkyl, lower alkoxy or halogen; in a compound of formula I* it being possible for the two substituents C₆H₄-R₁ and R₂ to be attached to each of the saturated carbon atoms of the saturated ring, either both to the same carbon atom or both to different carbon atoms; or of a stereoisomer or mixture of stereoisomers, or of a pharmaceutically acceptable salt thereof.

3. Use according to claim 1 of a compound of formula I, wherein
R₁ is lower alkyl; lower alkyl which is substituted by hydroxy, amino, di-lower alkylamino, 1 to 5 fluorine atoms, carboxy, lower alkoxycarbonyl, carbamoyl or cyano; or is nitro, halogen, hydroxy, lower alkoxy, amino, lower alkylamino, di-lower alkylamino, sulfo, sulfamoyl, formyl, iminomethyl; iminomethyl which is substituted at the nitrogen by hydroxy, lower alkoxy, lower alkanoyloxy, lower alkyl or phenyl; or is carboxy, lower alkoxycarbonyl, carbamoyl, lower alkylcarbamoyl, di-lower alkylcarbamoyl or cyano; and
R₂ is hydrogen, lower alkyl, lower alkoxy or halogen; or of a compound of formula I* wherein
n is 1, 2 or 3;
R₁ is as defined above for formula I; and
R₂ is hydrogen, lower alkyl, phenyl-lower alkyl, carboxy-lower alkyl, lower alkoxycarbonyl-lower alkyl, halogen, lower alkoxy, lower alkylthio, phenyl-lower alkylthio, phenylthio, carboxy, lower alkoxycarbonyl or lower alkanoyl;
in a compound of formula I* it being possible for the two substituents C₆H₄-R₁ and R₂ to be attached to each of the saturated carbon atoms of the saturated ring, either both to the same carbon atom or both to different carbon atoms; or of a stereoisomer or mixture of stereoisomers, or of a pharmaceutically acceptable salt thereof.

4. Use according to claim 1 of a compound of formula I, wherein
R₁ is lower alkyl, hydroxy-lower alkyl, halogen, amino, formyl, carboxy, lower alkoxycarbonyl, carbamoyl, N-lower alkylcarbamoyl or cyano; and
R₂ is hydrogen;
or of a compound of formula I*, wherein
n is 1, 2 or 3;
R₁ is as defined above for formula I; and
R₂ is hydrogen, lower alkylthio, lower alkoxycarbonyl, phenyl-lower alkyl, carboxy-lower alkyl or lower alkoxycarbonyl-lower alkyl;
in a compound of formula I* it being possible for the two substituents C₆H₄-R₁ and R₂ to be attached to each of the saturated carbon atoms of the saturated ring, either both to the same carbon atom or both to different carbon atoms; or of a stereoisomer or mixture of stereoisomers, or of a pharmaceutically acceptable salt thereof.

5. Use according to claim 1 of 5-(p-cyanophenyl)-5,6,7,8-tetrahydroimidaso[1,5-a]pyridine, or of a pharmaceutically acceptable non-toxic acid addition salt thereof.

6. Use according to claim 1 of (+)-5-(p-cyanophenyl)-5,6,7,8-tetrahydroimidaso[1,5-a]pyridine, or of a pharmaceutically acceptable non-toxic acid addition salt thereof.

7. Use according to claim 1 of (-)-5-(p-cyanophenyl)-5,6,7,8-tetrahydroimidaso[1,5-a]pyridine, or of a pharmaceutically acceptable non-toxic acid addition salt thereof.

## Revendications

1. Utilisation des composés de formule I 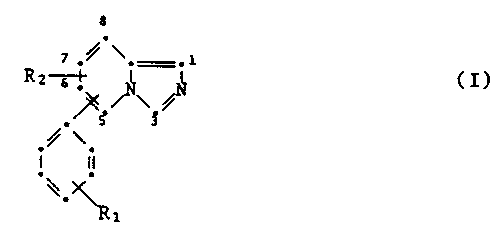 où R₁ représente l'hydrogène, un alkyle inférieur, un alkyle inférieur substitué, le nitro, un halogène, un hydroxy libre, éthérifié ou estérifié, un mercapto libre, éthérifié, éthérifié oxydé ou estérifié, un amino non substitué, mono- ou disubstitué, l'ammonio, un sulfo libre ou fonctionnellement modifié, un formyle libre ou fonctionnellement modifié, un acyle en C₂-C₂₀ ou un carboxy libre ou fonctionnellement modifié; et R₂ représente l'hydrogène, un alkyle inférieur, un alkyle inférieur substitué, un halogène; un hydroxy libre, éthérifié ou estérifié; un mercapto libre, éthérifié, éthérifié oxydé ou estérifié; un carboxy libre ou fonctionnellement modifié ou un acyle; de leurs dérivés 7,8-dihydrogénés; et des composés de formule I* 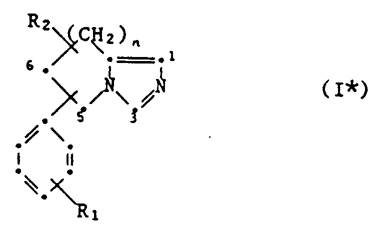 où n est égal à 0, 1, 2, 3 ou 4 et R₁ et R₂ sont définis comme ci-dessus pour la formule I; les deux substituants C₆H₄-R₁ et R₂ pouvant etre liés à chacun des atomes de carbone saturés du cycle saturé, ces deux substituants pouvant être soit liés au même atome de carbone, soit liés à des atomes de carbone différents; de leurs stéréoisomères, des mélanges de ces stéréoisomères et de leurs sels pharmaceutiquement utilisables pour la préparation de compositions pharmaceutiques pour le traitement de l'hyperaldostéronisme.

2. Utilisation selon la revendication 1, caractérisée en ce que l'on utilise les composés de formule I où R₁ représente l'hydrogène, un alkyle inférieur, un alkyle inférieur substitué par un hydroxy, un alcoxy inférieur, un alcanoyle inférieur oxy, un alcanoyle inférieur, un amino, un alkyle inférieur amino, un dialkyle inférieur amino, un halogène, un sulfo, un carboxy, un alcoxy inférieur carbonyle, un carbamoyle ou le cyano; le nitro, un halogène, l'hydroxy, un alcoxy inférieur, un alcanoyle inférieur oxy, un phénylsulfonyloxy, un alkyle inférieur sulfonyloxy, un mercapto, un alkyle inférieur thio, un alkyle inférieur sulfinyle, un alkyle inférieur sulfonyle, un alcanoyle inférieur thio, un amino, un alkyle inférieur amino, un dialkyle inférieur amino, un alkylène inférieur amino, le N-morpholino, le N-thiomorpholino, le N-pipérazino éventuellement substitué en position 4 par un alkyle inférieur, un trialkyle inférieur ammonio, un sulfo, un alcoxy inférieur sulfonyle, le sulfamoyle, un alkyle inférieur sulfamoyle, un dialkyle inférieur sulfamoyle, le formyle; l'iminométhyle éventuellement substitué sur l'azote par un hydroxy, un alcoxy inférieur, un alcanoyle inférieur oxy, un alkyle inférieur, un phényle ou un amino; un alcanoyle en C₂-C₇, un benzoyle, un carboxy, un alcoxy inférieur carbonyle, un carbamoyle, un alkyle inférieur carbamoyle, un dialkyle inférieur carbamoyle, le cyano, le 5-tétrazolyle, le 4,5-dihydro-2-oxazolyle éventuellement substitué par un alkyle inférieur ou un hydroxycarbamoyle; et R₂ représente l'hydrogène, un alkyle inférieur, un phénylalkyle inférieur, un carbonylalkyle inférieur, un alcoxy inférieur carbonylalkyle inférieur, un halogène, un hydroxy, un alcoxy inférieur, un alcanoyle inférieur oxy, un mercapto, un alkyle inférieur thio, un phénylalkyle inférieur thio, un phénylthio, un alcanoyle inférieur thio, un carboxy, un alcoxy inférieur carbonyle ou un alcanoyle inférieur; leurs dérivés 7,8-dihydrogénés; ou les composés de formule I* où n est égal à 0, 1, 2, 3 ou 4; et R₁ et R₂ sont définis comme ci-dessus pour la formule I; le noyau phényle dans les restes phénylsulfonyloxy, phényliminométhyle, benzoyle, phénylalkyle inférieur, phénylalkyle inférieur thio et phénylthio pouvant être non substitué ou substitué par un alkyle inférieur, un alcoxy inférieur ou un halogène; dans un composé de formule I* les deux substituants C₆H₄-R₁ et R₂ pouvant être liés à chacun des atomes de carbone saturés du cycle saturé, les deux substituants pouvant être soit liés au même atome de carbone, soit liés à des atomes de carbone différents; leurs stéréoisomères, les mélanges de ces stéréoisomères ou leurs sels pharmaceutiquement utilisables.

3. Utilisation selon la revendication 1, caractérisée en ce que l'on utilise les composés de formule I où R₁ représente un alkyle inférieur; un alkyle inférieur substitué par un hydroxy, un amino, un dialkyle inférieur amino, 1 à 5 atomes de fluor, un carboxy, un alcoxy inférieur carbonyle, un carbamoyle ou le cyano; le nitro, un halogène, un hydroxy, un alcoxy inférieur, un amino, un alkyle inférieur amino, un dialkyie inférieur amino, le sulfo, le sulfamoyle, le formyle, l'iminométhyle; l'iminométhyle substitué sur l'azote par un hydroxy, un alcoxy inférieur, un alcanoyle inférieur oxy, un alkyle inférieur ou le phényle; un carboxy, un alcoxy inférieur carbonyle, un carbamoyle, un alkyle inférieur carbamoyle, un dialkyle inférieur carbamoyle ou le cyano; et R₂ représente l'hydrogène, un alkyle inférieur, un alcoxy inférieur ou un halogène; ou les composés de formule I* où n est égal à 1, 2 ou 3; R₁ est défini comme ci-dessus pour la formule I et R₂ représente l'hydrogène, un alkyle inférieur, un phénylalkyle inférieur, un carboxyalkyle inférieur, un alcoxy inférieur carbonylalkyle inférieur, un halogène, un alcoxy inférieur, un alkyle inférieur thio, un phénylalkyle inférieur thio, un phénylthio, un carboxy, un alcoxy inférieur carbonyle ou un alcanoyle inférieur; dans un composé de formule I* les deux substituants C₆H₄-R₁ et R₂ pouvant être liés à chacun des atomes de carbone saturés du cycle saturé, les deux substituants pouvant être soit liés au même atome de carbone, soit liés à des atomes de carbone différents; leurs stéréoisomères, les mélanges de ces stéréoisomères ou leurs sels thérapeutiquement utilisables.

4. Utilisation selon la revendication 1, caractérisée en ce que l'on utilise des composés de formule I où R₁ représente un alkyle inférieur, un hydroxyalkyle inférieur, un halogène, un amino, le formyle, le carboxy, un alcoxy inférieur carbonyle, le carbamoyle, un N-alkyle inférieur carbamoyle ou le cyano; et R₂ est l'hydrogène; ou les composés de formule I* où n est égal à 1, 2 ou 3; R₁ est défini comme ci-dessus pour la formule I et R₂ représente l'hydrogène, un alkyle inférieur thio, un alcoxy inférieur carbonyle, un phénylalkyle inférieur, un carboxyalkyle inférieur ou un alcoxy inférieur carbonylalkyle inférieur; dans un composé de formule I* les deux substituants C₆H₄-R₁ et R₂ pouvant être liés à chacun des atomes de carbone saturés du cycle saturé, les deux substituants étant soit liés au même atome de carbone, soit liés à des atomes de carbone différents; leurs stéréoisomères, les mélanges de ces stéréoisomères ou leurs sels pharmaceutiquement utilisables.

5. Utilisation selon la revendication 1, caractérisée en ce que l'on utilise la 5-(p-cyanophényl)-5,6,7,8-tétrahydroimidazo[1,5-a]pyridine ou l'un de ses sels d'addition d'acide non toxiques, pharmaceutiquement utilisables.

6. Utilisation selon la revendication 1, caractérisée en ce que l'on utilise la (+)-5-(p-cyanophényl)-5,6,7,8-tétrahydroimidazo[1,5-a]pyridine ou l'un de ses sels d'addition d'acide non toxiques, pharmaceutiquement utilisables.

7. Utilisation selon la revendication 1, caractérisée en ce que l'on utilise la (-)-5-(p-cyanophényl)-5,6,7,8-tétrahydroimidazo[1,5-a]pyridine ou l'un de ses sels d'addition d'acide non toxiques, pharmaceutiquement utilisables.
